# EUROPÄISCHE PATENTANMELDUNG

(11) **EP 1 439 394 A1**
(43) Veröffentlichungstag der Anmeldung: **21.07.2004**
(21) Anmeldenummer: 02028551.6
(22) Anmeldetag: 20.12.2002
(51) Int. Cl.: G01N 33/94, G01N 33/68

(54) **Verfahren und Vorrichtung zum Erstellen von ADME Profilen in der frühen Wirkstoffentwicklung**

(71) Anmelder: Graffinity Pharmaceuticals Aktiengesellschaft, 69120 Heidelberg (DE)
(72) Erfinder: Uhrig, Rainer, Dr., 69234 Dielheim (DE); Sekul, Renate, Dr., 68526 Ladenburg (DE); Junker, Hans-Dieter, Dr., 69115 Heidelberg (DE); Lawrence, Ann-Marie, 55129 Mainz (DE); Vogt, Sabine, 68753 Waghäusel (DE)
(74) Vertreter: Büchel, Edwin, Dr.

(57) **Zusammenfassung**

Gegenstand der Erfindung ist ein Verfahren zur Analyse der Interaktion zwischen Wirkstoffvorstufen und ADME relevanten Biomolekülen, die Schritte umfassend die Immobilisierung einer Vielzahl unterschiedlicher Wirkstoffvorstufen sowie ggf. von Standardliganden auf einem festen Träger; das Inkontaktbringen eines ADME relevanten Biomoleküls mit den Wirkstoffvorstufen sowie das Messen der Interaktion zwischen immobilisierten Wirkstoffvorstufen und ADME relevantem Biomolekül. Diese Daten können zur Klassifizierung der Wirkstoffvorstufen hinsichtlich ihrer ADME Eigenschaften sowie zur Erstellung eines ADME-Profils der Wirkstoffvorstufen verwendet werden.

## Beschreibung

Die vorliegende Erfindung betrifft ein Verfahren und eine Vorrichtung zur Erstellung von ADME Profilen in der frühen Wirkstoffentwicklung.

Die Wirksamkeit eines Wirkstoffs hängt nicht allein von der intrinsischen Aktivität des Wirkstoffes gegenüber seinem Zielprotein, sondern auch von seiner Absorption, seiner Distribution, seinem Metabolismus sowie seiner Exkretion im menschlichen Körper ab, den sogenannten "ADME Charakteristika" des Wirkstoffes. Schätzungsweise 50% der Wirkstoffkandidaten scheitern wegen ihrer schlechten Bioverfügbarkeit, welche in den unzureichenden ADME Charakteristika der Wirkstoffe begründet liegt (Li, A.P. (2001), Drug Discovery Today 6 (7), 357-366). Dies macht deutlich, dass für den klinischen Erfolg eines Wirkstoffes neben der Zielprotein spezifischen Effektivität des Wirkstoffes auch seine ADME Charakteristika berücksichtigt werden müssen.

Der Stoffwechsel eines Wirkstoffes bestimmt mehrere wichtige Eigenschaften eines Wirkstoffes: Wird ein Wirkstoff schnell metabolisiert, muss er häufiger oder in größerer Menge verabreicht werden, um eine therapeutisch wirksame Dosis im Plasma aufrechtzuerhalten. Ausserdem kann ein Metabolit toxische Eigenschaften aufweisen. Darüber hinaus kann ein Wirkstoff oder dessen Metabolite die Wirkung eines gleichzeitig verabreichten zweiten Wirkstoffes beeinflussen.

Die bedeutendsten am Metabolismus eines Wirkstoffs beteiligten Enzyme sind die Cytochrom P450 Enzyme. Das Cytochrom P450 (CYP) System besteht aus einer Superfamilie membrangebundener Oxygenasen mit vielfältigen Funktionen. Der CYP vermittelte Metabolismus bestimmt häufig die Verweildauer eines Wirkstoffes im Körper und kann daher verschiedene andere ADME Eigenschaften beeinflussen. So musste beispielsweise die Roche Holding AG ihr Medikament Posicor vom Markt nehmen, da dieses Wirkstoffwechselwirkungen auf der Ebene des CYP3A4 Stoffwechsels (einer CYP Isoform) verursachte (Crespi C.L. (1999), Current Opinion in Drug Discovery & Development 2, 15-19).

Die Entwicklung solcher zum Scheitern verurteilter Wirkstoffkandidaten ist daher unbedingt zu vermeiden. Solche Wirkstoffkandidaten durchlaufen häufig einen Großteil des Entwicklungsprozesses, ohne unerwünschte Eigenschaften zu zeigen, bis letztere teilweise erst in den abschliessenden Tests oder schlimmstenfalls wie im obengenannten Beispiel erst nach der Zulassung auftreten und die betreffenden Wirkstoffkandidaten oder Wirkstoffe aus der Entwicklung bzw. vom Markt genommen werden müssen. Zu diesem Zeitpunkt hat die Entwicklung solcher Wirkstoffkandidaten bereits jahrelange Arbeit und Kosten in Millionenhöhe verursacht.

Es wäre daher von grossem Vorteil, wenn bereits zu einem frühen Stadium der Wirkstoffentwicklung ADME Eigenschaften von Wirkstoffvorstufen ermittelt werden könnten und damit eine Voreinschätzung der Risiken, die bei einer Weiterentwicklung der jeweiligen Wirkstoffvorstufen möglicherweise auftreten, getroffen werden könnten.

Um einen ADME Assay im HTS Format durchführen zu können, sollte er folgenden Kriterien genügen: Er sollte komponentenunabhängig sein und die Datenerfassung sollte sehr schnell erfolgen. Diese Voraussetzungen erfüllen die verfügbaren ADME Assays jedoch nicht. So beinhalten die meisten Metabolismusassays beispielsweise die Quantifizierung einer spezifischen Verbindung, die zudem nur in geringer Konzentration vorliegt. Ausserdem sind die analytischen Methoden von der verwendeten Verbindung abhängig und beinhalten oft eine chromatographische Aufreinigung. Bei diesen Assays dauert die Datenerhebung Stunden bis zu Tagen und kann daher nicht zur Verwendung im HTS herangezogen werden.

So werden beispielsweise zur Bestimmung der metabolischen Stabilität von Wirkstoffkandidaten gegenwärtig häufig Lebermikrosomen oder intakte Hepatocyten eingesetzt (Li, A.P. (2001), DDT 6 (7), 357-366). Lebermikrosomen werden durch Homogenisation und anschliessender Zentrifugation von Lebergewebe gewonnen. Nachteilig an der Verwendung von Mikrosomen ist, dass diese nur zur Evaluierung der Phase I Oxidation eingesetzt werden können, da die cytosolischen Phase II Enzyme durch das Präparationsverfahren verlorengehen.

Vorteilhafter ist daher ein Verfahren, mit dem sowohl Phase I und Phase II Enzyme analysiert werden können. Dies ist in Aktivitätsassays mit intakten Hepatocyten möglich. Nachteilig an den Hepatocyten-Assays ist jedoch, dass das Verfahren zur Gewinnung von für Metabolismus-Assays geeigneten Hepatocyten sehr aufwendig ist. Hierfür muss eine frisch isolierte Leber zuerst mit einer isotonen Pufferlösung, danach mit einer Collagenase-Lösung perfundiert werden. Die so gewonnenen Leberzellen können in Suspension oder als Monolayer für Aktivitätsassays eingesetzt werden.

Zur Bestimmung der metabolischen Stabilität eines Wirkstoffkandidaten wird dieser zusammen mit Hepatozyten oder Mikrosomen in einer Kulturschale, deren Boden von einer porösen Membran gebildet wird, inkubiert. Bei Verwendung von Mikrosomen müssen zusätzlich Enzym-Cofaktoren zugegeben werden. Nach einer Inkubationszeit von 0.5 bis 4 h wird ein organisches Lösungsmittel (z.B. Acetonitril) zugegeben, um die Reaktion zu stoppen und die Testkomponenten zu extrahieren. Die Kulturschale wird zentrifugiert, um das Reaktionsgemisch in eine neue Kulturschale zu filtrieren, in der das Reaktionsgemisch mittels chromatographischen und massenspektroskopischen Methoden (z.B. LC-MS), analysiert wird.

Zur Analyse von inhibitorischen oder induktiven Wirkstoffinteraktionen sind verschiedene Assays etabliert worden (Li, A.P. et al. (1999), Chem. Biol. Interact. 121, 17-35).

Bei den Inhibitionsassays werden primäre Hepatocyten oder Mikrosomen mit einem Wirkstoffkandidaten sowie verschiedenen Isoform-spezifischen Substraten wie z.B. Coumarin (CYP2A6), Dextromethorphan (CYP2D6) oder Testosteron (CYP3A4) inkubiert. Die Aktivität der spezifischen CYP Isoformen, welche durch die Bildung von Metaboliten messbar ist, wird durch HPLC oder LC-MS quantifiziert.

Nachteilig an Microsomen und Hepatozyten basierten Metabolismusassays ist, dass aufwendige Präparations-, Inkubations- und Analyseschritte notwendig sind, die sehr zeitaufwendig sind. Ausserdem ist von Nachteil, dass humane Mikrosomen und Hepatocyten nur in sehr geringen Mengen zur Verfügung stehen und daher oft auf andere Spezies zurückgegriffen werden muss. Hierbei können jedoch Spezies spezifische Unterschiede auftreten. Ein weiterer Nachteil dieser Zell-oder Organellen-basierten Assays ist, dass die Reproduzierbarkeit der Ergebnisse nicht immer gewährleistet ist.

Ein grosser Nachteil der obengenannten Assays ist ausserdem, dass sie nur sehr begrenzt parallelisierbar sind: Assays mit Hepatozyten-Monolayer werden zur Zeit in 24er Multiwellplatten durchgeführt, Assays unter Verwendung von Mikrosomen oder Hepatozyten in Lösung maximal in 96er Multiwellplatten. Nachteilig ist ausserdem, dass die Reaktionsprodukte aufgereinigt werden müssen. Diese Nachteile erschweren eine Verwendung von Mikrosomen oder Hepatozyten basierten Metabolismusassays im Hochdurchsatz (HTS) sehr.

Aufgrund der geringen Parallelisierbarkeit der verfügbaren Assays werden ADME Charakteristika gegenwärtig erst zu einem relativ späten Zeitpunkt der Wirkstoffentwicklung erfasst, zu dem die grosse Zahl der potentiellen Wirkstoffkandidaten durch Selektions- und Screeningprozesse bereits sehr stark eingeschränkt wurde. Dies kann jedoch wie bereits oben ausgeführt zu zeitaufwendigen und kostenintensiven Fehlentwicklungen in der Wirkstoffforschung führen.

Ein Ansatz, einen höheren Durchsatz zu erzielen, besteht darin, das experimentelle Design der bestehenden Assays zu reduzieren. So sind mittlerweile zell- und organellenfreie Metabolismusassays verfügbar.

Die am besten etablierten zellfreien Systeme arbeiten mit rekombinanten Enzymen, die unter Zusatz von Coenzymen und Cofaktoren meistens in einem gekoppelten Enzymassay verwendet werden (US 6,207,404; US 6,319,678). Hierfür wird ein fluorogenes Substrat eingesetzt. Die Zunahme des bei der Reaktion entstehenden Fluorophors wird als Maß für die Enzymaktivität angesehen. Bei Inhibitionsassays mit zu testenden Wirkstoffen kann so die Verminderung der Enzymaktiviät gemessen werden. Die Vorteile dieser Assays liegen darin, daß sie gut standardisierbar und reproduzierbar sind. Zudem sind sie gut parallelisierbar (96er Mikrotiterplattenformat). Nachteilig ist die Notwendigkeit, dass für jedes neue Enzym ein neues fluorogenes Substrat entwickelt werden muß und daß es teilweise große Schwierigkeiten bereitet, spezifische Substrate für das betreffende Enzym zu finden. Ein weiterer Nachteil dieser Assays ist, dass hierfür Coenzyme und Cofaktoren zugesetzt werden müssen.

Nachteilig an allen bisher beschriebenen Inhibitionsassays ist weiterhin, daß weder die bindende noch die gebundene Substanz in der Interaktion von Interesse detektiert werden können. Die Notwendigkeit, zu indirekten Detektionssystemen zu greifen und nicht markierungsfrei arbeiten zu können, sind hierbei die Hauptnachteile.

Nachteilig an den obengenannten ADME Verfahren ist weiterhin, dass für jedes ADME Kriterium (Absorption, Distribution, Metabolismus, Exkretion) ein separater und sehr aufwendiger Assay durchgeführt werden muss. Es wäre daher von grossem Vorteil, wenn Assays für verschiedene ADME Eigenschaften standardisiert durchgeführt werden könnten.

Ein Verfahren, bei dem die Interaktion zwischen einem Wirkstoff und ADME relevanten Biomolekülen untersucht wird, wird in WO00/79268 beschrieben. Hierbei wird vorteilhaft ein Festphasenassay verwendet. Das Festphasenscreening allgemein stellt eine wichtige Verbesserung im HTS dar, da hier auf relativ einfache Weise eine Automation und damit eine Parallelisierung der einzelnen Prozesse sowie eine Miniaturisierung des Assaysystems erreicht werden kann.

In WO00/79268 werden ADME relevante Biomoleküle auf einem Träger unter Verwendung eines organischen Films, bestehend aus einer selbstassemblierenden Monolage (SAM) auf einer Goldoberfläche immobilisiert. Zur Assaydurchführung werden bekannte Wirkstoffe im Durchfluss über die Trägeroberfläche geleitet. Vorteilhaft wird als Detektionsmethode die Oberflächenplasmonenresonanz (SPR) eingesetzt, die ohne Markierung der Wirkstoffe oder ADME relevanten Biomolekülen arbeiten kann und somit einen direkten Nachweis der Interaktion von Wirkstoffen und analysierten ADME relevanten Biomolekülen mittels einer allgemein anwendbaren Methode ermöglicht.

Zur Ermittlung einer ADME Eigenschaft werden verschiedene Konzentrationen des Wirkstoffes eingesetzt und die Bindung an das jeweilige ADME relevante Biomolekül gemessen. Daraus wird eine Gleichgewichtskonstante (K_{D}-Wert) zwischen Wirkstoffkandidaten und ADME relevantem Biomolekülen berechnet. Die in Gleichgewichtskonstanten (K_{D}-Werten) angegebenen Bindungsdaten werden zur Ermittlung einer ADME Eigenschaft mit Literaturdaten verglichen.

Nachteilig ist, dass viele Wirkstoffkandidaten nur bedingt wasserlöslich sind und daher mit dem in WO00/79268 beschriebenen Verfahren nur sehr schwierig und aufwendig zu testen sind, da die Flusszelle nur sehr geringe Lösungsmittelkonzentrationen (z.B. DMSO) toleriert.

Ein weiterer Nachteil ist, dass das für die Bestimmung der ADME Charakteristika verwendete Protein immobilisiert wird. Dies kann einerseits die Bindungseigenschaften des Proteins beeinträchtigen. Ausserdem führt es zu technischen Problemen mit niedermolekularen Wirkstoffkandidaten, da am Detektionslimit gearbeitet wird. Nachteilig ist weiterhin, dass die verwendete Sensoroberfläche ein Hydrogel enthält, was zu zu einer hohen unspezifischen Proteinbindung führen kann.

Das in WO 00/79268 beschriebene Verfahren ist ungeeignet für ein ADME Profiling von Hunderten von Wirkstoffvorstufen, da die zu analysierenden, immobilisierten Verbindungen sequentiell gemessen werden und hierfür maximal 4 Kanäle zur Verfügung stehen.

Ausserdem ist die Bestimmung von K_{D} Werten vergleichsweise aufwendig und nur schwer zu parallelisieren.

Überraschenderweise wurde nun gefunden, dass die in einem Festphasenscreening gewonnenen Bindungsdaten von Wirkstoffvorstufen an ein ADME relevantes Biomolekül zum Ermitteln einer ADME Eigenschaften dieser Wirkstoffvorstufen verwendet werden können. Dabei reicht es aus, jeweils nur eine einzige Konzentration des ADME relevanten Biomoleküls einzusetzen und nur einen einzigen Bindungswert zu bestimmen, was den Einsatz des erfindungsgemässen Verfahrens im Hochdurchsatz ermöglicht. Je nach verwendetem ADME relevanten Biomolekül ermöglicht dies, die Absorptions-, Distributions-, Metabolismus- oder Exkretionseigenschaften der jeweiligen Wirkstoffvorstufe einzuschätzen. Ein grosser Vorteil dabei ist, dass sowohl für die Einschätzung der Absorptions-, der Distributions-, Metabolismus und Exkretionseigenschaften derselbe Assay verwendet werden kann und damit die aufwendige Assayentwicklung entfällt. Dadurch ist es mit dem erfindungsgemässen Verfahren möglich, in kurzer Zeit nicht nur einen, sondern eine Vielzahl von ADME Eigenschaften zu ermitteln und somit im Hochdurchsatz ADME Profile von Wirkstoffvorstufen zu erstellen.

Die in dem erfindungsgemässen Verfahren gewonnenen Informationen dienen dazu, Wirkstoffvorstufen mit ungünstigen ADME Eigenschaften bereits zu einem sehr frühen Stadium der Wirkstoffentwicklung zu erkennen und diese entweder
- vollständig von der weiteren Wirkstoffentwicklung auszuschliessen, wenn es sich um Wirkstoffvorstufen handelt, deren ADME Profile sehr viele potentielle Schwachstellen aufweisen; oder
- bei einer ausreichend großen Anzahl von identifizierten "Hits" mit vielversprechendenm Bindungsverhalten zunächst zugunsten der Alternativen zurückzustellen; oder
- zumindest bezüglich ihrer vermeintlichen Schwachstellen eingeschätzt werden können und somit schon in einem sehr frühen Stadium das Hauptaugenmerk auf diese Schwachstellen gelenkt werden kann. Dadurch könnten schon frühzeitig Nachfolgeuntersuchungen (z.B. funktionelle Assay) eingeleitet werden, um das Ausmaß der zu erwartenden Probleme abzuschätzen.
Darüber hinaus sollten Wirkstoffvorstufen, die keinen der obigen Fälle betreffen, uneingeschränkt weiter entwickelt werden können.

Der Erfindung liegt somit die Aufgabe zugrunde, ein HTS fähiges Verfahren und Meßsystem zum Ermitteln von ADME Eigenschaften und zum Erstellen von ADME Profilen in einer frühen Phase der Wirkstoffentwicklung bereitzustellen.

Gegenstand der Erfindung ist ein Verfahren zur Analyse der Interaktion zwischen Wirkstoffvorstufen mit ADME relevanten Biomolekülen, die Schritte umfassend
(a) Immobilisierung einer Vielzahl von Wirkstoffvorstufen auf einem festen Träger in einem zweidimensionalen Array;
(b) Inkontaktbringen der Wirkstoffvorstufen mit einem ADME relevanten Biomolekül;
(c) Bestimmung der Bindungsstärken zwischen immobilisierten Wirkstoffvorstufen und ADME relevantem Biomolekül;
(d) Klassifizierung der Wirkstoffvorstufen nach ihrer Bindungsstärke und Zuordnung einer ADME Eigenschaft.

Weiterhin ist Gegenstand der Erfindung ein Meßsystem umfassend
(a) einen festen Träger auf dem eine Vielzahl von unterschiedlichen Wirkstoffvorstufen in einem zweidimensionalen Array immobilisiert sind,
(b) ein an mindestens einer Wirkstoffvorstufe gebundenes ADME relevantes Biomolekül und
(c) einen Detektor.

Das erfindungsgemäße Verfahren kann zusätzlich den Schritt umfassen:
(d) Wiederholung von Schritt (b) bis (d) mit unterschiedlichen ADME relevanten Biomolekülen, um ein ADME Profil der Wirkstoffvorstufen zu erstellen.

Bevorzugt beruht im erfindungsgemäßen Verfahren die Bestimmung der Bindungsstärken auf Gleichgewichtsmessungen. Von Vorteil ist, daß für die Messungen zur Bestimmung der Bindungsstärken die Wirkstoffvorstufen und ADME relevanten Biomoleküle jeweils nur in einer einzigen Konzentration bereitgestellt werden muß.

Zur Validierung des erfindungsgemässen Verfahrens wurden Bindungsdaten von Wirkstoffkandidaten, welche mit dem erfindungsgemässen Verfahren gemessen wurden, mit einem vorher bestimmten Wirkstoff-Korrelationsdiagramm verglichen (s. Beispiel 2). Das Wirkstoff-Korrelationsdiagramm repräsentiert aus der Literatur bekannte oder in einem funktionellen Assay ermittelte Affinitätsdaten von Wirkstoffkandidaten an das jeweilige ADME relevante Biomolekül. Der Vergleich ergab eine hohe Übereinstimmung der im Festphasenscreening gewonnenen Bindungsdaten mit Literaturdaten bzw. den in Lösung durchgeführten Assays.

Die Immobilisierung einer Vielzahl von Wirkstoffvorstufen ermöglicht den Zugang zu einem in hohem Masse parallelisierten und miniaturisierten HTS Verfahren: Mit dem erfindungsgemässen Verfahren kann die Bindung eines ADME relevanten Biomoleküls an mehreren Hunderten und Tausenden von Wirkstoffvorstufen gleichzeitig gemessen werden. Dies wird durch den Einsatz eines vorteilhaften zweidimensionalen Probenarrays sowie eines parallelen Detektionssystems ermöglicht.

Darüber hinaus ermöglicht die Inkorporation von internen Standards auf dem Festphasenträger eine Normierung und Klassifizierung der Messdaten.

Zudem ist es vorteilhaft, daß bei der Detektion die direkte Bindung zwischen potentieller Wirkstoffvorstufe und ADME relevantem Biomolekül nachgewiesen wird.

Ausserdem ist es möglich, mit dem erfindungsgemässen Festphasenassay metabolische Enzyme ohne Cofaktoren im HTS zu testen, wobei die Bindung im Festphasenscreening der Aktivität im Lösungsassay entspricht. Vorteilhaft am erfindungsgemässen Verfahren ist weiterhin, dass metabolische Enzyme sowohl der Phase I als auch der Phase II getestet werden können. Zudem ist es nicht notwendig, für jedes neue Enzym eine neue Methode bzw. einen neuen fluorogenen Farbstoff entwickeln zu müssen. Nur die Screeningbedingungen müssen gegebenenfalls an das entsprechende Enzym angepaßt werden.

Zur Klassifizierung von Wirkstoffvorstufen und der Zuordnung einer ADME Eigenschaft zu diesen Verbindungen stehen mehrere Methoden zur Verfügung. Es kann eine Klasseneinteilung anhand bekannter Wirkstoffe erfolgen. Hierzu wird die Bindungsstärke dieser zu einem ADME relevanten Biomolekül ermittelt. Diese bilden dann jeweils Repräsentanten einer Klasse. Fällt die Bindungsstärke einer Wirkstofforstufe ebenfalls in diese Klasse, kann angenommen werden, daß diese eine mit diesem ADME-Molekül zusammenhängende ADME-Eigenschaft des bekannten Wirkstoffs vergleichbare ADME-Eigenschaft besitzt.

Weiterhin ist es möglich eine Klassifizierung der Wirkstoffvorstufen dadurch zu erhalten, daß eine Normierung aller ermittelter Bindungsstärken beispielsweise auf den stärksten Wert erfolgt. Aufgrund der Bindungsstärke zu einem ADME relevanten Biomolekül kann dann eine Zuordnung zu einer für dieses Biomolekül charakteristischen Eigenschaft erfolgen. Insbesondere bei der Verwendung interner Standards kann auf eine Normierung verzichtet werden. Generell kann eine Klassifizierung anhand der Bindungsstärke zu einem ADME relevanten Molekül direkt durch Vergleich mit anderen Werten erfolgen. Die Zuordnung zur entsprechenden ADME Eigenschaft erfolgt jeweils über das ADME relevante Biomolekül.

Ein ADME Profil ergibt sich, wenn mindestens zwei unterschiedliche ADME relevante Biomoleküle herangezogen werden. Vorzugsweise werden mindestens vier ADME relevante Biomoleküle herangezogen die vorzugsweise jeweils aus dem Bereich Adsorption, Distribution, Metobolismus bzw. Exkretion stammen.

Mit dem erfindungsgemässen Verfahren ist es somit möglich, eine Einschätzung der ADME Eigenschaften von Wirkstoffvorstufen zu gewinnen. Damit ist es möglich, die ADME Eigenschaften von Verbindungen bereits zu einem sehr frühen Stadium der Wirkstoffentwicklung einzuschätzen und je nach Sachlage (siehe oben) Folgerungen für deren Weiterentwicklung abzuleiten.

Unter dem Begriff "Wirkstoffvorstufen" werden im Rahmen der Erfindung Verbindungen einer chemischen Bibliothek, welche als Ausgangsverbindungen bei der Wirkstoffentwicklung dienen, sowie Wirkstofffragmente ,"Hits" oder Leitstrukturen verstanden.
Unter dem Begriff "Wirkstofffragmente" werden im Rahmen der Erfindung Moleküle verstanden, die als Teilstruktur in einem Wirkstoff enthalten sein können, aber noch nicht ausreichend charakterisiert sind, um als Leitstruktur eingestuft zu werden.
Unter dem Begriff "Hits" werden im Rahmen der Erfindung Moleküle verstanden, die in einem Primärscreen eine klare Interaktion mit dem Zielmolekül, dem sog. Target, zeigen und deren Struktur bekannt ist.
Unter dem Begriff "Leitstrukturen" werden im Rahmen der Erfindung Verbindungen mit bekannter biologischer Wirkung verstanden, deren Struktur systematisch mit Hilfe eines standardisierten Herstellungsverfahrens optimiert wird.
Unter einem Wirkstoff wird im Rahmen der Erfindung eine Substanz verstanden, die zur Behandlung von Krankheiten bei Menschen oder Tieren eingesetzt werden kann. Ein Wirkstoff kann in einem medizinische Diagnoseverfahren oder zur Wiederherstellung, Korrektur oder Veränderung von physiologischen Funktionen eingesetzt werden.

Bevorzugte Wirkstoffvorstufen umfassen Peptide, Oligonucleotide, Kohlenhydrate (Glycoside), Isoprenoide, Enzyme, Lipidstrukturen und organische Moleküle.

Ganz besonders bevorzugt sind kleine organische Moleküle (small molecules oder small molecular weight molecules), welche häufig Leitstrukturen oder Startpunkt für die Weiterentwicklung zu Leitstrukturen für Wirkstoffe oder auch Fragmente derselben sind.

In der Literatur stellt zumeist das Molekulargewicht die Definitionsgrundlage für solche kleinen Moleküle dar. In WO 89/03041 und WO 89/03042 werden Moleküle mit Molekülmassen von bis 7000 g/mol als kleine Moleküle beschrieben. Üblicherweise werden jedoch Molekülmassen zwischen 50 und 3000 g/mol, häufiger aber zwischen 75 und 2000 g/mol und meistens im Bereich zwischen 100-1000 g/mol angegeben. Als Beispiele sei auf die Schriften WO 86/02736, WO 97/31269, US-A-5928868, US-A-5242902, US-A-5468651, US-A-5547853, US-A-5616562, US-A-5641690, US-A-4956303 und US-A-5928643 verwiesen. Im Rahmen der vorliegenden Erfindung gelten Wirkstoffvorstufen mit einem Molekulargewicht unter 3000, bevorzugt unter 1000, am meisten bevorzugt unter 750 g/mol als kleine organische Moleküle.

Um den Durchsatz bei der Ermittlung einer ADME Eigenschaft erhöhen zu können, ist es vorteilhaft, eine möglichst große Anzahl an Wirkstoffvorstufen zu immobilisieren. Hierbei kann eine Anzahl von mindestens 5, bevorzugt mindestens 10, besonders bevorzugt mindestens 100, ganz besonders bevorzugt 1000, am meisten bevorzugt mindestens 5000 verschiedenen Wirkstoffvorstufen immobilisiert werden.
Weiterhin muß berücksichtigt werden, daß zur Immobilisierung der Wirkstoffvorstufen verschiedene Stellen der Wirkstoffvorstufe benutzt werden können. Somit erhält die Wirkstoffvorstufe auf der Oberfläche eine andere Orientierung und kann mitunter ein anderes Bindungsverhalten zeigen. Erfindungsgemäß werden vereinfachend auch unterschiedliche Orientierungen einer Wirkstoffvorstufe als "unterschiedliche" Wirkstoffvorstufen verstanden.

Im erfindungsgemässen Verfahren ist es möglich, die Identität einer jeden Wirkstoffvorstufe durch den Ort der Immobilisierung zu bestimmen. Hierfür ist eine Anordnung der Wirkstoffvorstufen in einem zweidimensionalen Raster (Array) günstig, da dadurch die Identität einer jeden Wirkstoffvorstufe durch deren x- und y-Koordinaten festgelegt ist.
Bevorzugt ist daher auch, dass im erfindungsgemäßen Verfahren und Meßsystem ein planarer fester Träger zum Einsatz kommt. Hierbei kann der Träger ein oder mehrere Teile umfassen, wobei diese Materialien umfassen, ausgewählt aus der Gruppe bestehend aus Glas, Kunststoff, natürliche oder synthetische Membran, Metall, Metalloxid und Nichtmetalloxid.

Im Rahmen des erfindungsgemässen Verfahrens bzw. Meßsystems wird die Interaktion zwischen Wirkstoffvorstufen und ADME relevanten Biomolekülen ermittelt. Hierzu werden die auf dem Array immobilisierten Wirkstoffvorstufen mit einen ADME relevanten Protein in Lösung in Kontakt gebracht. Als "ADME relevante Biomoleküle" werden Biomoleküle verstanden, über deren Interaktion mit Wirkstoffvorstufen eine ADME Eigenschaft für die Wirkstoffvorstufen ermittelt werden kann.

Der Begriff "Absorption" bezieht sich im Rahmen der Erfindung auf die Aufnahme eines Wirkstoffes von seinem Applikationsort in die systemische Zirkulation. Ein oral applizierter Wirkstoff muss beispielsweise mehrere Membranbarrieren entlang des gastrointestinalen Traktes passieren. Wie gut ein Wirkstoff Membranen passiert, ist daher ein wichtiger Parameter, um die Absorption und Distribution des Wirkstoffes in verschiedene Organe und Gewebe zu beurteilen. Die Absorption in die systemische Zirkulation kann über passive Diffusion durch Zellen hindurch (transzellulärer Transport), zwischen Zellen über zelluläre Verbindungen (parazellulärer Transport) oder mittels Transporterproteinen (aktiver Transport) erfolgen.
Wie hierin offenbart, können die Absorptionseigenschaften einer Wirkstoffvorstufe aus Daten eines Festphasenscreenings gewonnen werden, bei dem die Bindung eines oder einer Vielzahl von immobilisierten Wirkstoffvorstufen an ein geeignetes Biomolekül, vorzugsweise ein Liposom oder ein Transporterprotein analysiert wird.

Hierfür geeignete Liposomen beinhalten vorzugsweise organische Verbindungen, ausgewählt aus Glycerophospholipiden, Glyceroglycolipiden, Sphingophospholipiden und Sphingoglycolipiden, Phosphatidylcholinen, Phosphatidyletanolaminen, Phosphatidylserinen, Phosphatidylglycerol, Phosphatidylsäure, Phosphatidylinositol, Galactopyranosid, Digalactopyranosid, Ceramid-Phosphatidylcholin, Ceramid-Phosphatidyletanolamin, Ceramid-Phosphatidylserin, Ceramid-Phosphatidylglycerol, CeramidePhosphatidylsäure, Ceramide-Phosphatidylinositol, Sphingomyelin, Glucosylceramiden, Glucocerebrosiden, Galactoceramiden, Galactocerebrosiden, Gangliosiden, Cardiolipin, Cholesterol, Lanosterol, Ergosterol, Stigmasterol, Sitosterol, Diacetylphosphat, Bolaamphiphile, Polyglycerolmonoalkylether, sowie Liposom-bildende Moleküle ausgewählt aus amphiphilen Polymeren, Aminosäuren, Kronenether und Di(acyloxy-)dialkylsilane.
Darüberhinaus sind alle Proteine, die an aktiven Transportprozessen beteiligt sind, geeignete Biomoleküle zur Ermittlung der Absorptionsparameter von Wirkstoffvorstufen.

Der Begriff "Distribution" bezieht sich im Rahmen der Erfindung auf die Verteilung des Wirkstoffes über die systemische Zirkulation in das Zielgewebe im Organismus. Wie hierin offenbart, können die Distributionseigenschaften von Wirkstoffvorstufen aus Daten eines Festphasenscreenings gewonnen werden, bei dem die Bindung eines oder einer Vielzahl von immobilisierten Wirkstoffvorstufen gegen ein geeignetes Biomolekül, vorzugsweise ein Plasmaprotein, ein Liposom oder ein Transporterprotein analysiert wird.

Geeignete Plasmaproteine zur Charakterisierung der Distributionseigenschaften von potentiellen Wirkstoffvorstufen sind beispielsweise Immunoglobulin G (7s-y-Globulin), IgG; Immunoglobulin A, IgA; Sekretorisches IgA, sIgA; Immunoglobulin M (19s-y-Globulin) IgM; Immunoglobulin D, IgD; Immunoglobulin E, IgE; α1-Antitrypsin, α1-P1, α1-A; α1-Antichymotrypsin, (α1X-Glycoprotein) α1-X; Inter-α-Trypsininhibitor, α1-TI; Antithrombin III, (Heparin Cofaktor) AT III; α-Thiol Proteinaseinhibitor (LMW Kininogen) αTPI; C1-Inaktivator, C1 Esterase-Inhibitor (α2-Neuraminoglycoprotein) C1 INA; α2-Makroglobulin, α2-M; α2-Antiplasmin, α2-AP; Cystatin C (Post-y-Globulin; y-Trace Protein); C1q (11S Protein); C1r; C1s; C2; C3 (β1C-Globulin), C4 (β1E-Globulin); C5 (β1F-Globulin); C6; C7; C8; C9; Faktor B, (C3-Proaktivator; β2-Glycoprotein II; Glycinreiches P-Glycoprotein); Faktor D (C3-Proaktivator Convertase); Properdin, P; Factor I, (C3b Inaktivator); C4-bindendes Protein; Fibrinogen, FI, Prothrombin, F II; Factor V (Proaccelerin); FV; Factor VII, (Proconvertin), F VII; Factor VIII (antihämophiler Faktor) F VIII; Factor VIII-Verwandtes Antigen; FV III; Rag (von Willebrand Faktor) (VWF); Faktor IX ('Christmas' Faktor) F IX; Faktor X, (Stuart-Power Faktor) F X; Factor XI (Plasma Thromboplastin Antecedens) F XI; Faktor XII (Hageman Faktor) F XII; Faktor XIII (Fibrin stabilisierender Faktor) F XIII; hochmolekulares (HMW) Kininogen (Fitzgerald Faktor); Prekallikrein (Fletcher Faktor); Plasminogen; Protein C; Protein S; Albumin, ALB; Haptoglobin, HP, Hp 1-1, Hp 2-1, Hp 2-2; Prealbumin (Transthyretin, Thyroxin bindendes Prealbumin); Retinol-bindendes Protein RBP; Thyroxinbindendes Globulin TBG; Transcortin (Corticosteroid-bindendes Globulin) CBG; Sex-Hormon-bindendes Globulin (Steroid-bindendes β Globulin) SHBG; Vitamin D-bindendes Protein (Gc-Globulin) VDBP; Transcobalamin I, TC I; Trancobalamin II, TC II; Transferrin (Siderophilin) TF; Ferritin; Hämopexin, HPX; Apolipoprotein A, Apo A-1, Apo A-II; Apolipoprotein B, Apo B-48, ApoB-100; Apolipoprotein C; Apo C-1, Apo C-II, Apo C-III; Apolipoprotein E, Apo E; Apolipoprotein (a), apo (a); Serum Amyloid A, SAA; α-Fetoprotein, AFP; Ceruloplasmin, CP; Serum Amyloid P Protein (9.5S α1-Glycoprotein; α1-Makroglobulin) SAP; α2-HS-Glycoprotein, α2 HS; Fibronectin FN, C-reaktives Protein, CRP; β2-Mikroglobulin; α1-Mikroglobulin, α1-M.
Besonders bevorzugte Plasmapropteine zur Ermittlung von Distributionseigenschaften einer Wirkstoffvorstufe sind Humanes Serum Albumin (HSA), Alpha-1-Acid Glycoprotein (AAG) und Lipoproteine, insbesondere Very Low Density Lipoprotein (VLDL), Intermediate Density Lipoprotein (IDL), Low Density Lipoprotein (LDL), High Density Lipoprotein (HDL) und Chylomikronen sowie Chylomikronen-Remnants.

Der Begriff "Metabolismus" bezieht sich auf alle chemischen Reaktionen zur Biotransformation von endogenen und exogenene Substanzen, die in einer Zelle stattfinden. Typischerweise wird ein Wirkstoff zunächst oxidiert (Phase I Oxidation). Anschliessend erfolgt die Konjugation von hochpolaren Molekülen wie beispielsweise Glucuronsäure, Sulfat, Methionin, Cystein oder Glutathion an die oxidierte Gruppe (Phase II Konjugation).
Wie hierin offengelegt, können Metabolismuseigenschaften einer Wirkstoffvorstufe aus Daten eines Festphasenscreenings gewonnen werden, bei dem die Bindung eines oder einer Vielzahl von immobilisierten Wirkstoffvorstufen gegen ein geeignetes metabolisches Biomolekül analysiert wird. Vorteilhaft am erfindungsgemässen metabolischen Assay ist, dass damit sowohl Phase I als auch Phase II Metabolismuseigenschaften bestimmt werden können, was mit vielen herkömmlichen Assays nicht möglich ist.

Geeignete Biomoleküle zur Ermittlung der Metabolismuseigenschaften von Wirkstoffvorstufen sind beispielsweise Glutathion-Thioether, Butyrylcholinesterase, humane Serum Paraoxonase/Arylesterase, N-Acetyltransferase, UDP-Glucuronosyltransferase (UDPGT) Isoenzyme, Glutathion-S-Transferasen (GSTs), GST1, GST2, GST3, GST4, GST5, GST6, Alkohol Dehydrogenase (ADH), ADH I, ADH II, ADH III, Aldehydehydrogenasen(ALDHs), cytosolische ALDHs, mitochondriale ALDHs, Monoaminoxidasen, Flavin-enthaltende Monoaminoxidasen, Superoxid-Dismutase (SOD), Catalase, Amidasen, Thioester, Carboanhydrasen, S-Actin, Mercaptide, Cholinesterase, lysosomale Carboxypeptidase, Calpain, Retinol Dehydrogenase, Retinyl Reduktase, Folat Hydrolase, Protein Phosphatasen, Carboxyesterase, Endopeptidasen, Enterokinasen, Neutrale Endopeptidasen, Carboxypeptidases, Dipeptidyl Carboxypeptidasen (Peptidyl-Dipeptidase), Angiotensin-umwandelndes Enzym, G-Glutamyl Transpeptidase E.C.2.3.2.2, Carboxypeptidase P, Folat Konjugase, Dipeptidasen, Glutathion Dipeptidasen, Membran-Gly-Leu-Peptidasen, Asp-Leu-Dipeptidase, Enterozytische intrazelluläre Peptidasen, Amino Tripeptidase E.C.3.4.11.4, Amino Dipeptidase E.C.3.4.13.2, Prodipeptidase, Arg-selektive Endoproteinase, Hydrolasen, Endopeptidase-24.11, Endopeptidase-2, Dipeptidyl Peptidase IV, Membran Dipeptidase GPI, Glycosidasen, Sucrase-Isomaltase, Lactase-Glycosyl-Ceraminidase, Glucoamylase-Maltase, Trehalase, Carbohydrase Enzyme, alfa-Amylase (pankreatisch), Disaccharidasen, Lactase-Phlorizin Hydrolase, Epoxid-Hydrolase (mEH), Glucoamylase, Lactase-Glycosyl-Ceramidase, Xanthin Oxidase, NADPH Oxidase, Aminoxidase, Aldehydoxidase, Dihydroorotat Dehydrogenase, Peroxidasen, humane pankreatische exokrine Enzyme, Trypsinogen 1, Trypsinogen 2, Trypsinogen 3, Chymotrypsinogen, Proelastase 1, Proelastase 2, Kallikreinogen, Procarboxypeptidase A1, Procarboxypeptidase A2, Procarboxypeptidase B 1, Procarboxypeptidase B2, Glycosidase, Amylase, Lipasen, Triglyceridlipase, Collipase, Carboxylester Hydrolase, Phospholipase A2, Nucleasen, DNAse I, Ribonucleotid Reductasen, A1 Amylase 1, A2 Amylase 2, Carboxylester Lipase, Prophospholipase A, Trypsinogen 1, Trypsinogen 2, Trypsinogen 3, Trypsinogen 4, Chymotrypsinogen 1, Chymotrypsinogen 2, Procarboxypeptidase A1, Procarboxypeptidase A2, Procarboxypeptidase B1, Procarboxypeptidase B2, Ribonuclease, Lithostatin, 4-nitrophenol UDP-Glucuronyltransferase (UDPGT) Isoenzyme, 4-Nitrophenol UDPGT, 17b-Hydroxysteriod UDPGT, 3-a-Hydroxysteroid UDPGT, Billirubin UDPGT, Phenol UDPGT, 5-Hydroxytryptamin UDPGT, 4-Hydroxybiphenyl UDPGT, Peptidasen, Aminopeptidasen, Aminopeptidase N, Aminopeptidase A, Aminopeptidase P, Dipeptidyl Peptidasen, Dipeptidyl Peptidase IV, b-Casomorphin, Carboxypeptidase P Angiotensin I, Angiotensin II, Endopeptidase-24.11, Endopeptidase-24.18, Substanz P, Exopeptidasen, Aminopeptidase A (EC 3.4.11.7), Aminopeptidase P (EC 3.4.11.9), Aminopeptidase W (EC 3.4.11.-), Dipeptidyl Peptidase IV (EC 3.4.14.5), g-Glutamyl transpeptidase (EC 2.3.2.2), Carboxypeptidase P (EC 3.4.17.), Carboxypeptidase M (EC 3.4.17.12), microsomale Dipeptidasen, Gly-Leu Peptidase, Endopeptidase, Endopeptidase-24.11 (EC 3.4.24.11), Endopeptidase-2 (EC 3.4.24.18), Meprin, Endopeptidase-3, microsomale Leukotrien C4 Synthase, UDP-Glucuronosyltransferase (UGT) Isozymes, pankreatische Enzyme, Elastase, Aminopeptidasen, Chymotrypsin, Trypsin, Carboxypeptidase A, Methyltransferasen, O-Methyltransferasen, N-Methyltransferasen, S-Methyltransferasen, Catechol-O-Methyltransferasen, Sulfotransferasen, Mg2+-ATPase, Rezeptoren für Wachstumsfaktoren, Rezeptor-Tyrosinkinasen, Alkalische Phosphatasen, ATPasen, Na, K-ATPase, Ca2+-ATPase, Leucin Aminopeptidase, K+Kanäle.

Bevorzugte Biomoleküle zur Ermittlung von Phase I Metabolismuseigenschaften von Wirkstoffvorstufen sind Isoforme der Cytochrom P450 (CYP) Familie, beinhaltend CYP1A2, CYP2A1, 2A2, 2A3, 2A4, 2A5, CYP2B1, 2B2, 2B3, 2B4, 2B5, CYP2C1, 2C2, 2C3, 2C4, 2C5, 2C6, 2C7, 2C10, 2C11, 2C12, CYP2D1, 2D2, 2D3, 2D4, 2D5, CYP3A1, 3A2, 3A3, 3A5, 3A7, CYP4A1, 4A2, 4A3, 4A4, CYP4A11, CYP P450 11A (P450scc), CYP P450 17 (P45017a), CYP P450 19 (P450arom), CYP P450 51 (P45014a), CYP P450 105A1, CYP P450 105B1. Besonders bevorzugt sind CYP1A1, CYP1A12, CYP2A6, CYP1B1, CYP2B6, CYP2B7, CYP2C8, CYP2C9, 2C18, 2C19, CYP2E1, 3A5, 3A7, 4A11, 4F2, 4F3A, 4F3B, 19. Ganz besonders bevorzugt sind CYP3A4, CYP2D6 und die CYP2C-Enzyme, da diese Isoformen für ca. 95% des Metabolismus bekannter Wirkstoffe verantwortlich sind. Eine weitere Klasse von Enzymen, die am Phase I Metabolismus beteiligt sind, sind die Flavinmonooxygenasen (FMO), insbesondere FMO1-5.

Bevorzugte Biomoleküle zur Ermittlung von Phase II Metabolismuseigenschaften von Wirkstoffvorstufen sind die UDP-abhängige Glucuronosyl Transferase (UGT) und Isoenzyme, UGT1A1, 3, 4, 6, 7, 8, 9,10, UGT2B7, UGT2B15, die Glutathion-S-Transferase und Isoenzyme (GST), GST A1-1 Alpha, GST A2-2 Alpha, GST M1a-1a Mu, GST M1b-1b Mu, GST M2-2 Mu, GST M3-3 Mu, GST M4-4 Mu, GST M5-5 Mu, GST P 1-1 Pi, GST T1-1 Theta, GST T2-2 Theta, Sulfotransferasen (ST), ST 1a1*2, ST1A2*1, ST1A3, ST1E, ST2A1, Phenol-Sulfo-Transferase (PST) und Östrogen-Sulfo-Transferase (EST) sowie deren Isoformen.

Der Begriff "Exkretion" bezieht sich im Rahmen der Erfindung auf die Ausscheidung des Wirkstoffes aus dem Organismus. Die Exkretion eines Wirkstoffes kann sowohl über passive Diffusion als auch aktiv mittels spezifischer Transporterproteine erfolgen.
Wie hierin offenbart, können Exkretionseigenschaften einer Wirkstoffvorstufe aus Daten eines Festphasenscreenings gewonnen werden, bei dem die Bindung eines oder einer Vielzahl von immobilisierten Wirkstoffvorstufen gegen ein geeignetes Biomolekül, vorzugsweise ein Liposom oder ein Transporterprotein analysiert wird.

Geeignete Transporterproteine zur Ermittlung von Exkretionseigenschaften (sowie eines Absorptions-und/oder Distributionsparameters) beinhalten Glukose-Transportproteine (GLUT), GLUT 1, GLUT 1-5, GLUT-2, Neutrale Aminosäuretransporter, kationische Aminosäuretransporter, Dipeptide/H+-Kotransport, Gallensäuretransporter, ABC Transporter, Prostaglandintranspoprter, Na/H+-Antiporter, Natrium-Phosphat-Kotransporter, Na-Sulfat-Kotransporter, Neurotransmittertransporter, Na/Cl-Kotransporter, Norepinephrin Na/Cl-Kotransporter, Dopamin Na/Cl-Kotransporter, Serotonin Na/Cl-Kotransporter, Glutamat Na+-Kotransporter, P Glycoprotein, Cl-Kanäle, CFTR abhängige CI-Kanäle, Antigenpeptid-Transporter, TAP1, TAP2, Resistenzprotein der Lunge (LRP), mrp (Maus), C. elegans mrp1 (Nematode), C. elegans mrp2 (Nematode), YCF1 (Hefe), YOR1/YRS1(Hefe), Sulfonylharnstoff-Rezeptor (SUR), SUR1 (human), sur2 (Ratte, Maus), Neutrale Aminoräuretransporter, Monoglyceride, L-Lysophosphatidylcholine, Transportproteine für Bilirubin, Signalrezeptoren, ATPasen, Na-abhängiger/unabhängiger Gallensäuretransporter, Cl-Kanäle, Na/H-Kanäle, GS Konjugat Transporter, Dipolare Aminosäuretransporter, Folattransporter, Na-K-ATPase, Nukleosidtransporter, Mitoxantrontransporter (MXR1, MXR2); Intestinale Oligopeptidtransporter, PEPT1, renale Oligopeptidetransporter, PEPT2, 'Breast Cancer Resistance Protein' (BCRP).

Besonders bevorzugt sind Multidrug Resistance Proteins (MDRs) und Multidrug Resistance associated Proteins (MRPs), Multidrug Resistance Protein 1 (MDR1, P-Glycoprotein), Multidrug Resistance Protein 2 (MDR2), Multidrug Resistance Protein 3 (MDR3); Multidrug Resistance associated Protein 1 (MRP1), Multidrug Resistance associated Protein 2 (MRP2, cMOAT, cMRP), Multidrug Resistance associated Protein 3 (MRP3, MOAT-D, cMOAT-2), Multidrug Resistance associated Protein 4 (MRP4, MOAT-B), Multidrug Resistance associated Protein 5 (MRP, MOAT-C, pABC11), Multidrug Resistance associated Protein 6 (MRP6, MOAT-E, MLP-1, ARA), Organische Kationentransporter (OCT), OCT1-3, Organische Anionentransporter (OAT), OAT1-3, OATP A-F, OCTN1-2, Bilitranslokase (BTL), sowie Pumpen für den Export von Gallesalzen (BSEP, SPGP).

Mittels eines geeigneten Detektionsverfahren wird die Affinität der auf dem Array immobilisierten Wirkstoffvorstufen an ein in Lösung befindliches, ADME relevantes Biomolekül bestimmt. Insbesondere, um ADME Eigenschaften einer Vielzahl von Wirkstoffvorstufen erstellen zu können, ist der Detektionsschritt sowie dessen Vorbereitung möglichst schnell und parallel durchzuführen. Das erfindungsgemäße Meßsystem sollte vorteilhafterweise ebenfalls diese Bedingungen erfüllen. Dies fördert auch die Vergleichbarkeit der einzelnen Meßergebnisse untereinander. Dabei ist es günstig, wenn alle Wirkstoffvorstufen nahezu gleichzeitig mit dem ADME relevanten Biomolekül in Kontakt kommen. Weiterhin ist es vorteilhaft, daß bei der Detektion die direkte Bindung zwischen Wirkstoffvorstufen und ADME relevantem Biomolekül nachgewiesen wird. Ebenso sollte der Detektor zur parallelen Erfassung aller Bindungsereignisse in der Lage sein und die Detektion selbst sollte parallel erfolgen. Vorteilhafterweise ist der Detektor eine CCD-Kamera. Weiterhin ist es vorteilhaft, wenn die Detektionsmethode markierungsfrei erfolgt. Prinzipiell können Detektionsverfahren auf radioaktiven, optischen oder elektrischen Verfahren beruhen. Bevorzugt sind hierbei reflektionsoptische Verfahren wie die Oberflächenplasmonen Resonanz (SPR).

Wird SPR als Detektionsmethode eingesetzt, ist es vorteilhaft, zunächst eine Messung des Microarrays ohne Lösung (Luftmessung) durchzuführen und danach eine Messung des Microarrays mit der Pufferlösung durchzuführen, in der anschließend das ADME relevante Biomolekül gelöst wird (Puffermessung). Anschliessend wird die Pufferlösung gegen eine Lösung des ADME relevanten Biomoleküls in demselben Puffer ausgetauscht und nochmals gemessen (Proteinmessung). Die Puffermessungen und die Proteinmessungen werden mittels der Luftmessung normiert und anschließend wird die Differenz zwischen den beiden Minima der Proteinmessung und der Puffermessung ermittelt.

Die so ermittelte Verschiebung (in nm) zwischen den beiden (ggf. normierten) Messungen ist ein Maß für die Bindungsstärke zwischen den immobilisierten Liganden und dem ADME relevanten Biomolekül (s. Beispiel 1).

Insbesondere beim Einsatz von reflektionsoptischen Methoden ist es von Vorteil, wenn der Träger mindestens zwei Teile umfaßt, wobei ein Teil aus zumindest teilweise lichtdurchlässigem Material und das andere eine Metallschicht sein kann. Insbesondere kann es sich hierbei um einen Glaskörper und eine Goldschicht handeln. Bei dem Glaskörper kommen beispielsweise ein Prisma oder eine Glasplatte in Frage. Bevorzugt ist der Träger zur Oberflächenplasmonen Resonanz (SPR) geeignet. Ein geeignetes Sensorsystem ist beispielsweise in WO-A-01/63256 offenbart, auf dessen Offenbarungsgehalt hiermit vollinhaltlich Bezug genommen wird.

Die Wirkstoffvorstufen werden anhand ihrer gemessenen Bindungsstärke an das ADME relevante Protein in verschiedene Klassen eingeteilt. Die Zugehörigkeit zu einer Klasse stellt die ADME Eigenschaft der Wirkstoffvorstufe dar.

Das erfindungsgemässe Verfahren ermöglicht es, Verbindungen mit ungünstigen ADME Eigenschaften, d.h. Verbindungen, die eine hohe Bindungsaffinitäten an eines oder mehrere ADME relevante Biomoleküle zeigen, bereits zu einem sehr frühen Stadium der Wirkstoffentwicklung zu erkennen und von einer Weiterentwicklung auszuschliessen oder deren potentiellen Schwachstellen aufzudecken. Verbindungen, die keine oder nur eine sehr schwache Bindung sowohl an Absorptions-, Distributions-, Metabolismus- sowie Exkretionsrelevante Biomoleküle, zeigen, werden als günstig für eine Weiterentwicklung eingeschätzt.

Insbesondere um ein ADME Profil der Wirkstoffvorstufen zu erstellen, ist es vorteilhaft, eine relative Bindungsstärke zu bestimmen. Diese ergibt sich aus dem Quotient der gefundenen Bindungsstärke der jeweiligen Wirkstoffvorstufe und der Bindungsstärke des jeweils stärksten gefundenen Binders für das entsprechende ADME relevante Biomolekül. Die relativen Bindungsstärken der Wirkstoffvorstufen an verschiedenen ADME relevante Biomoleküle werden nebeneinander dargestellt und das so erhaltene Profil zeigt auf einen Blick, für welche ADME relevanten Biomoleküle starke Bindung erhalten wurde und weist somit auf die vermeintlichen Risiken bei der Weiterentwicklung als Wirkstoff hin.

Im erfindungsgemässen Verfahren können neben den Wirkstoffvorstufen zudem bekannte Wirkstoffe als sogenannte Standardliganden auf dem Array inkorporiert werden, die zur internen Normierung der Messwerte dienen. Die Standardliganden dienen zur Klassifizierung der Wirkstoffvorstufen anhand ihrer Affinitätsdaten in sehr schwache, schwache, mittlere, starke und sehr starke Binder. Vorteilhaft an diesen internen Standards ist, dass die Daten besser, da vergleichbarer sind, als wenn die Standardliganden sequentiell gemessen werden, wie dies in dem in der Patentschrift W0 00/79268 beschriebenen Verfahren erfolgt.

Als Standardliganden geeignet sind bekannte Wirkstoffe oder Derivate derselben, für die pharmakokinetische Daten aus der Literatur und aus funktionellen Assays in Lösung vorhanden sind. Weiterhin müssen die Standardliganden eine Funktionalität zur Ankopplung an die Oberfläche enthalten, wobei die Kopplungsstelle nicht an der Interaktion zwischen dem zu screenenden Biomolekül und dem Standardliganden beteiligt sein darf.
Geeignete Standardliganden für die Bestimmung von Distributionsparametern sind beispielsweise Atenolol (sehr schwach), Chinin (schwach), Chinidin (schwach), Propranolol über Esterfunktion gekoppelt (mittel), Desimpramin (mittel), Nortriptylin (stark), Warfarin (sehr stark). Für die Bestimmung von Metabolismusparametern eignen sich zum Beispiel Atenolol (sehr schwach), Desimpramin (schwach), Nortriptylin (schwach), Perphenazin, (mittel), Progesteron (sehr stark).

Ein weiterer Vorteil des erfindungsgemässen Verfahrens und Meßsystems ist, dass nicht das ADME relevante Biomolekül, sondern die Wirkstoffvorstufen immobilisiert werden. Kommt es zu einer Interaktion, entsteht durch die Bindung des in Lösung befindlichen ADME relevanten Biomoleküls ein sehr viel stärkeres Signal als wenn die Wirkstoffvorstufe in Lösung ist, wie dies in dem in der Patentschrift W000/79268 beschriebenen Verfahren der Fall ist. Dies ist insbesondere vorteilhaft, wenn die Wirkstoffvorstufen kleine organische Moleküle sind.

Die Immobilisierung der Wirkstoffvorstufen und gegebenenfalls der Standardliganden kann direkt oder indirekt auf dem Träger erfolgen. Es gibt mehrere Möglichkeiten der Anbindung von Molekülen an eine feste Oberfläche. Hierbei sind beispielhaft eine kovalente, ionische oder adsorptive Anbindung zu nennen. Das Molekül kann unverändert oder chemisch modifiziert eingesetzt werden. Chemische Modifikation umfaßt das Verändern vorhandener Reaktivitäten, etwa das Aktivieren vorhandener funktioneller Gruppen oder das Hinzufügen eines weiteren Moleküls, das die direkte oder indirekte Anknüpfung an die Oberfläche ermöglicht. Hierzu können einfache Additions- oder Substitutionsreaktionen dienen.

Um häufig auftretende ungewünschte unspezifische Anbindung der Wirkstoffvorstufen an die Oberfläche des Trägers, insbesondere wenn dieser aus einer Kunststoff- oder Metalloberfläche besteht, zu vermeiden oder zu vermindern, ist eine organische Zwischenschicht vorteilhaft.
Häufig wird hier eine selbstassemblierende Monoschicht (SAM) verwendet, die eine Adsorption des Wirkstoffvorstufen auf dem Träger vermeidet. Die Selbstorganisation zu einem dichten Film erfolgt normalerweise durch hydrophobe Wechselwirkung langkettiger Kohlenwasserstoffe an deren einem Ende eine funktionelle Gruppe vorhanden ist, die die Anbindung an den Träger ermöglicht und an derem anderen Ende eine funktionelle Gruppe die Bindung der Wirkstoffvorstufen ermöglicht. Verbindungen, die diese funktionellen Bausteine umfassen (Kopf-, Fußgruppe, hydrophober Teil), werden auch Anker genannt. Weiterhin kann der Anker einen Spaceranteil besitzen, der bevorzugt Ethylenglycoleinheiten enthält, die eine geringe unspezifische Proteinadsorption gewährleisten. Im Stand der Technik wird häufig zusätzlich auf die organische Zwischenschicht ein Polymer, wie Streptavidin, aufgebracht. Wegen der möglichen unerwünschten Wechselwirkung zwischen diesem Polymer und dem Membranprotein ist eine polymerfreie Oberfläche bevorzugt.

Vorteilhafterweise werden zu den erwähnten Ankermolekülen noch sogenannte Verdünnermoleküle zugemischt, um die Konzentration auf der Oberfläche zu steuern. Eine zu dichte Oberflächenkonzentration kann durch sterische Hinderung nachteilig sein. Verdünnermoleküle sind strukturell den Ankermolekülen angepaßt, jedoch besitzen sie keine Kopfgruppe für die Anbindung der Wirkstoffvorstufen, da dieses vermieden werden soll. Weiterhin sind sie üblicherweise kürzer als die Ankermoleküle, um eine Beeinträchtigung der Erreichbarkeit der Wirkstoffvorstufen für das stabilisierte Membranprotein zu vermeiden.

Beispielsweise können SAMs durch Chemisorption von Alkylthiolen auf einer Metalloberfläche (z.B. Gold) erzeugt werden. Die langkettigen Moleküle packen sich als SAM auf die Festphase, wobei die Goldatome von den Schwefelfunktionen komplexiert werden. Ein weiteres Beispiel ist die Silanisierung von Glas oder Silizium mit reaktiven Epoxid- oder Aminogruppen-haltigen Silanen, anschließende Acylierung der Aminogruppen, beispielsweise mit Nukleosidderivaten (Maskos und Southern, Nucl. Acids Res. 20 (1992) 1679-84). Zur Synthese von Anker und Verdünnermolekülen sei auf WO 00/73796 A2 und DE-A-100 27 397 verwiesen, auf deren Offenbarungsgehalt hiermit vollinhaltlich Bezug genommen wird.

Das erfindungsgemäße Verfahren und Meßsystem können zur Klassifizierung oder zum Erstellung von ADME Profilen von Wirkstofffvorstufen im Hochdurchsatzscreening verwendet werden. Die daraus gewonnenen Informationen können zur Unterstützung bei der Selektion von Wirkstoffvorstufen für die weitere Wirkstoffentwicklung eingesetzt werden.

### Beispiel 1

### Festphasenscreening gegen CYP3A4, CYP2D6, HSA, AAG

Ein Goldchip, bevorzugt in den Abmaßen einer Mikrotiterplatte (12.4 cm x 8.2 cm), mit 4608 Sensorfeldern wurde mit einer 1:10 Mischung von Ankermolekül und Verdünnerkomponente in Ethylenglycol (EG) und 1% TFA inkubiert (Gesamtkonzentration 1.0 mM). Ankermolekül und Verdünnerkomponente wurden synthetisiert wie in Beispiel 11 von WO-A-01/02072 beschrieben. Anschließend wurde der Chip je dreifach in 1. Methanol/1%TFA, 2. H₂O/0,1% TFA und 3. H₂O/0,02M HOAc gewaschen sowie unter Stickstoffstrom getrocknet.

9216 Wirkstoffvorstufen (kleine organische Moleküle) wurden kommerziell erworben oder mittels kombinatorischer Festphasensynthese hergestellt. Alle Verbindungen weisen eine Carboxy-Funktionalität auf, die mit einem Amin umgesetzt wurden, das endständig eine Thiolgruppe zur Anbindung an die Maleimid-Kopfgruppe des Ankers trägt. Die mittlere Molekularmasse der freien Carbonsäuren beträgt 359 g/mol, wobei die kleinste Verbindung ein Molekulargewicht von 60 g/mol und die größte von 743 g/mol aufweist.

Auf zwei so vorbehandelten Chips wurde dann eine Bibliothek der 9216 modifizierten Wirkstoffvorstufen in entsprechenden Lösungen mit einem Pinning-Tool abgesetzt. Die Wirkstoffvorstufen, die so auf die Oberfläche gebracht werden, sind in einer 40 µM Lösung aus 0,2 M Phosphatpuffer (Pi), 5 mM EDTA und 20% (v/v) EG, pH 7.0, gelöst. Das Pinning Tool setzt ca. 10 nl pro Spot ab, so daß in jedem Spot ein hoher Überschuß des Wirkstoffvorstufen-Tag Konjugates gegenüber der oberflächengebundenen Maleimid-Gruppe gewährleistet wird und somit eine vollständige Belegung der Maleimid-Gruppen erreicht werden kann. In den freien Bereichen wurden die Maleimid-Gruppen anschließend durch Inkubation des Chips in 0,2 M Pi, pH 7.0, 10mM Mesh für 30 min abgesättigt. Danach wurde je dreimal mit 1. H₂O/0,02M HOAc, 2. MeOH/H₂O und 3. 2-Propanol gewaschen sowie unter Stickstoffstrom getrocknet.

Die Analyse potentieller Bindungspartner der ADME relevanten Biomoleküle CYP3A4, CYP2D6, HSA und AAG erfolgte anschließend mittels SPR-(Oberflächen-Plasmonen-Resonanz-) Analyse in einem SPR-Analysegerät wie in WO-A-01/63256 beschrieben. Die ADME relevanten Biomoleküle wurden in folgenden Konzentrationen in HBS-EP-Puffer ('BIA-certified', BR-1001-88, Bestandteile: 0,01 M HEPES pH 7,4; 0,15 M NaCl,; 3 mM EDTA; 0,005 % polysorbate 20(v/v) ) auf den Sensorchip gegeben: CYP2D6 (100 nM in HBS), CYP3A4 (100 nM in HBS), HSA (10 µM in HBS) und AAG (25 µM in HBS). Die Verschiebung der Wellenlänge wurde nach 120 min aufgezeichnet. Durch die eindeutige Zuordnung der (x,y)-Koordinate zu einer bestimmten Verbindung ist diese eindeutig identifizierbar. Wirkstoffvorstufen mit hohen Verschiebungswerten sind kritisch im Hinblick auf die weitere Wirkstoffentwicklung. Die Klassifizierung von zehn beispielhaft herausgegriffene Wirkstoffvorstufen aus diesem Beispiel sind in Beispiel 4 wiedergegeben.

### Beispiel 2

### Bestimmung einer Distributionseigenschaft

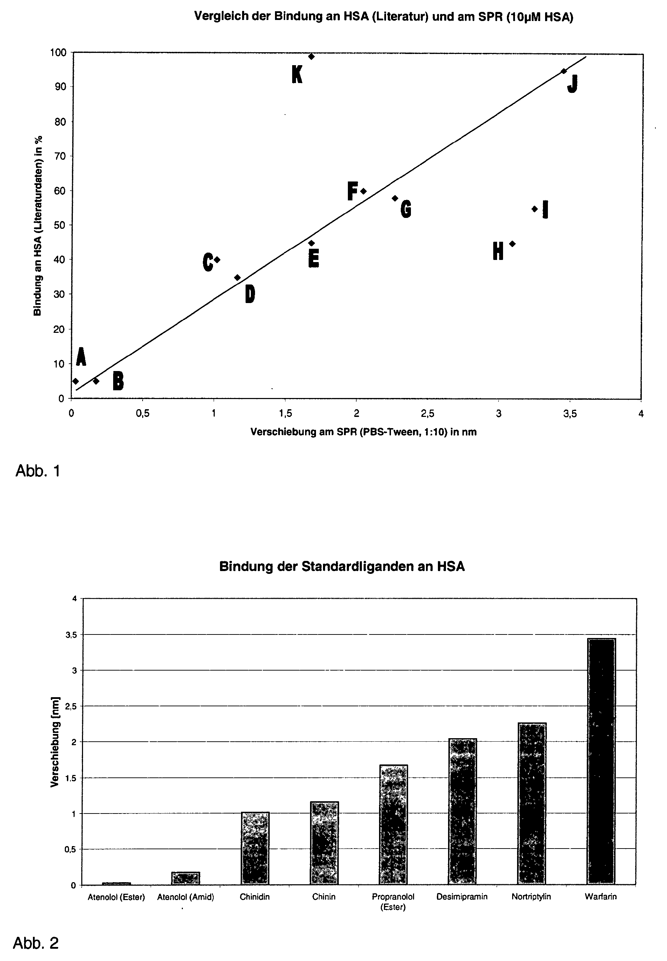

Zum Screening wurde eine 10 µM Lösung von HSA in PBS ohne Tween (2,5 ml) auf den Microarray (96er-Feld, 1536er-Format) gegeben, auf dem verschiedene Wirkstoffe und chemische Verbindungen immobilisiert worden waren. Das Festphasenscreening wurde durchgeführt wie in Beispiel 1 beschrieben.
Folgende Wirkstoffe wurden analysiert: A - Atenolol (als Ester gekoppelt); B - Atenolol (als Amid gekoppelt); C - Chinidin; D - Chinin; E - Propranolol (Ester); F - Desimipramin; G - Nortriptylin; H - Propranolol (Amid); I -Testosteron; J - Warfarin; K - Naproxen (Abb. 1).

Die Daten wurden mit Literaturdaten verglichen, bei denen die Proteinbindung mit klassischen Methoden bestimmt wurde und gegebenenfalls auf die Proteinbindung bei einer Wirkstoffkonzentration von 10 µM extrapoliert wurde (Pike, E. et al. (1981), Clin. Pharmacokinetics 6, 367-374; Brinkschulte, M. et al. (1980), Naunyn-Schmiederberg's Arch. Pharmacol. 314, 61-66; Pike, E. et al. (1982), Clin. Pharmacol Ther. 32(2), 228-34; Wanwimolruk, S. (1992), J Pharm. Pharmacol. 44(10), 806-11; Albani, F. (1984), Br. J. Clin. Pharmac. 18, 244-246; Ferrer, J.M. et al. (1998), J. Protein. Chem. 17(2), 115-119;Javaid, J.I. et al. (1983), Biochem. Pharmacol. 32(7), 1149-53).

Es zeigt sich eine gute Korrelation zwischen Literaturdaten und den ermittelten Verschiebungen. Anhand der bekannten Distributionseigenschaften der oben genannten Wirkstoffe, kann anhand der Bestimmung ihrer Bindungsstärke eine Klasseneinteilung (Klassifizierung) erfolgen, die wiederum eine Klassifizierung unbekannter Wirkstoffvorstufen erlaubt, so daß die ADME Eigenschaft der bekannten Wirkstoffe (Standardliganden) je nach Klassifizierung der entsprechenden Wirkstoffvorstufe zugeordnet werden kann.

Hieraus ergibt sich mit Hilfe der in Abb. 2 gezeigten Standardliganden eine Klassifizierung in sehr schwach (<0,7 nm), schwach (0,7-1,4 nm), mittel (1,4-2,1 nm), stark (2,1-2,8 nm) und sehr stark (>2,8 nm).

### Beispiel 3

### Bestimmung einer Metabolismuseigenschaft

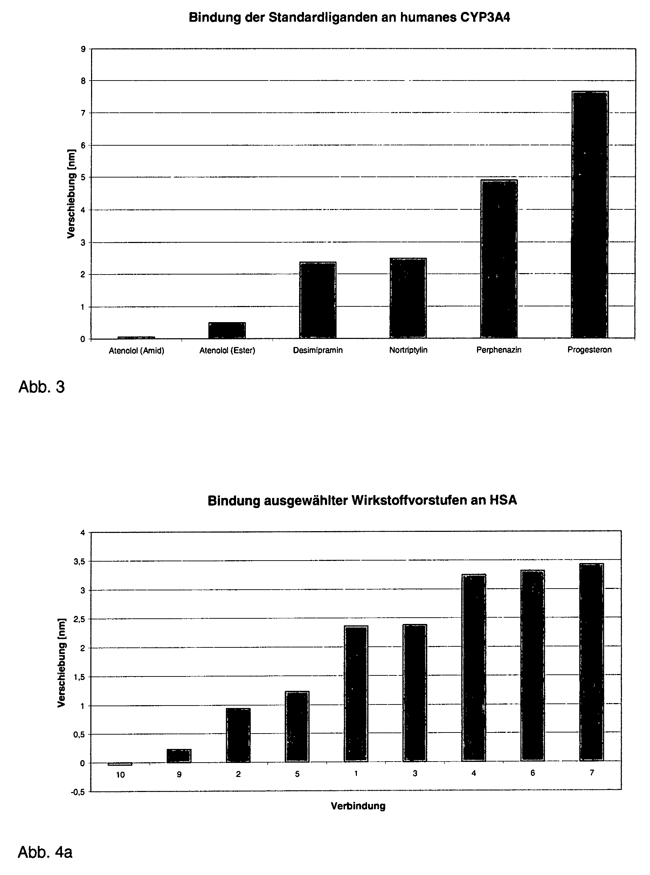

Analog zu Beispiel 2 wurde mit Hilfe der in Abb. 3 gezeigten Standardliganden und deren Festphasenscreening gegen CYP3A4 eine Klassifizierung in sehr schwach (<1,6 nm), schwach (1,6-3,2 nm), mittel (3,2-4,8 nm), stark (4,8-6,4 nm) und sehr stark (>6,4 nm) durchgeführt. Anhand der bekannten Metabolismuseigenschaften der Standardliganden ist eine Zuordnung dieser ADME Eigenschaft für nicht bekannte Wirkstoffvorstufen möglich.

### Beispiel 4

### Klassifizierung von Wirkstoffvorstufen

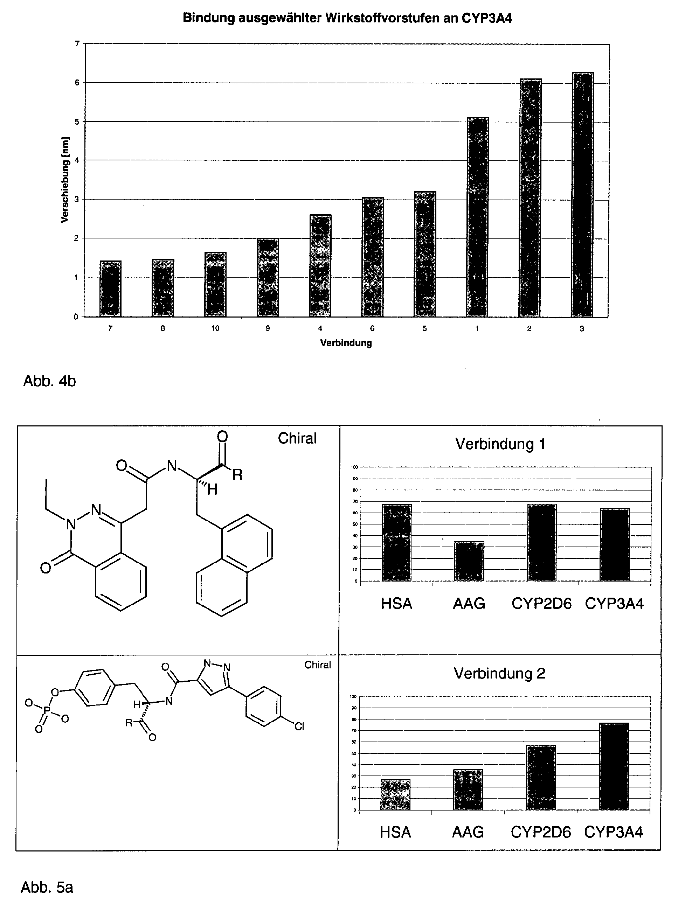

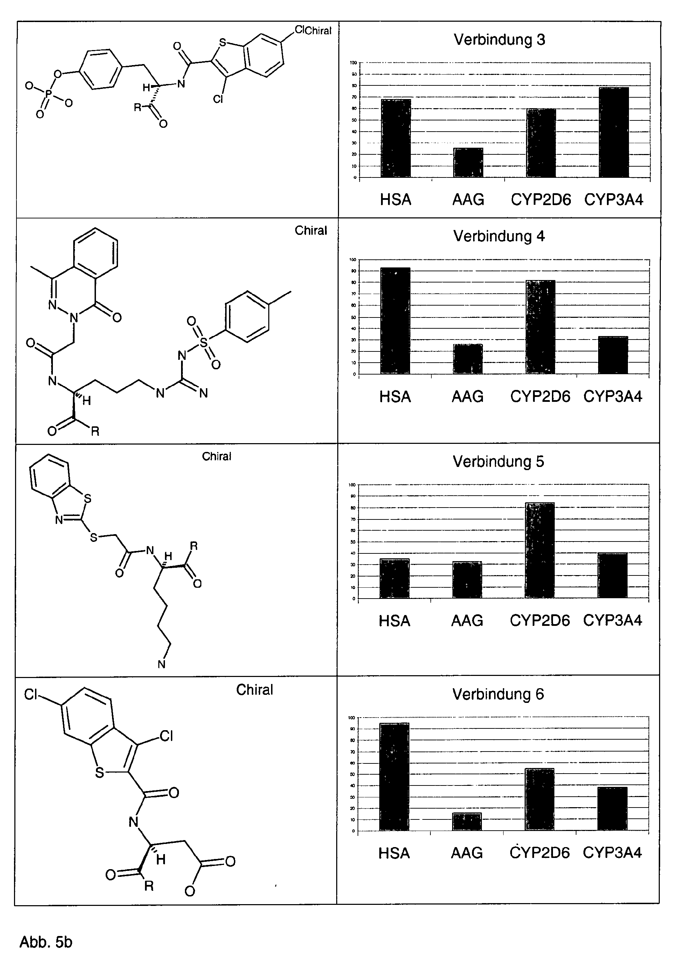

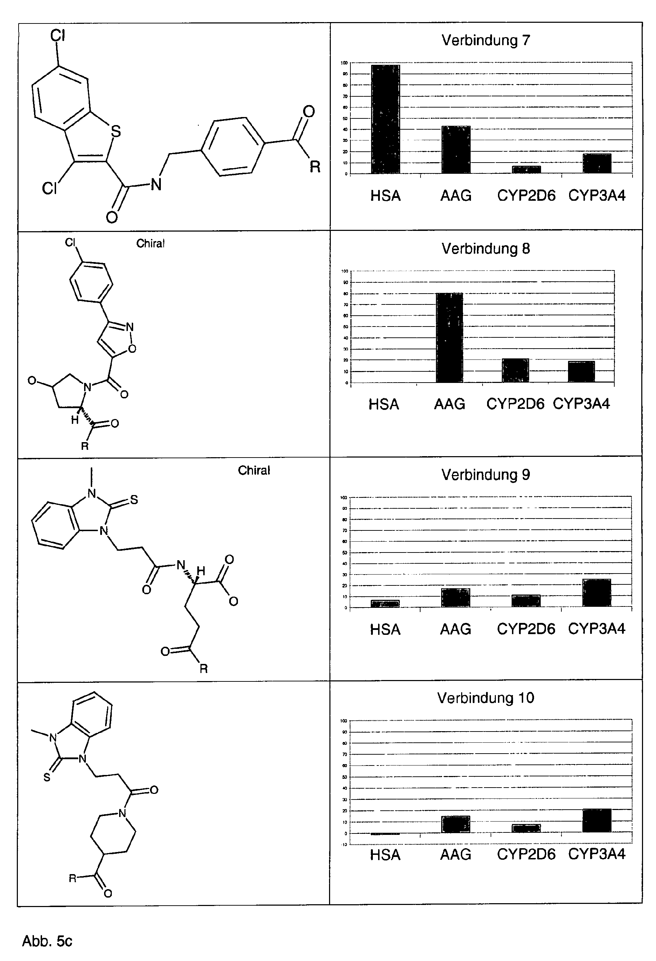

Anhand der Klasseneinteilung in Beispiel 2 und 3 kann für zehn exemplarische Verbindungen aus Beispiel 1, deren chemische Struktur in Abb. 5 eine Klassifizierung durchgeführt werden. Abb. 4 zeigt die ermittelten Verschiebungen, die eine direkte Einteilung für HSA (Abb. 4a) und CYP3A4 (Abb. 4b) von sehr schwach bis sehr stark erlauben. Für HSA ergibt sich, daß die Verbindungen 4, 6 und 7 (sehr starke Binder) und Verbindungen 1 und 3 (starke Binder) bezüglich ihrer Distributionseigenschaften kritisch im Hinblick auf eine Weiterentwicklung zu sehen sind. Die Verbindungen 2 und 5 (schwache Binder) und Verbindungen 9 und 10 (sehr schwache Binder) sind bezüglich ihrer Distributionseigenschaften als unbedenklich einzustufen.
Für CYP3A4 ergibt sich, daß die Verbindungen 1, 2 und 3 als starke Binder eingestuft werden können und sind damit bezüglich ihrer Metabolismuseigenschaften kritisch im Hinblick auf eine Weiterentwicklung zu sehen. Die Verbindung 5 wird als mittlerer Binder eingestuft. Um eine Aussage über die Metabolismuseigenschaften von Verbindung 5 zu treffen, müssten weitere Metabolismusassays durchgeführt werden. Verbindungen 4, 6, 9 und 10 (schwache Binder) und Verbindungen 7 und 8 (sehr schwache Binder) sind bezüglich ihrer Metabolismuseigenschaften als unbedenklich einzustufen.

### Beispiel 5

### ADME Profiling von Wirkstoffvorstufen

Mit dem erfindungsgemässen Verfahren wurden exemplarisch für zehn chemische Verbindungen ADME Profile erstellt (siehe Abb. 5). In Spalte 1 sind die Strukturen der Verbindungen abgebildet. der Rest R bezeichnet die Stelle, an der das omega-Mercaptoamin angebunden ist. In Spalte 2 sind die ADME-Profile für die untersuchten Proteine HSA, AAG, CYP2D6 und CYP3A4 gezeigt. Die Höhe der Balken entspricht der Bindungsaffinität der jeweiligen chemischen Verbindung gegenüber dem betreffenden ADME relevanten Protein. Die Bindungsaffinitäten sind als relative Werte angegeben, die die prozentuale Bindung im Verhältnis zum stärksten beobachteten Binder wiedergeben.

Die Verbindungen 1 und 3 zeigen gegen drei der vier analysierten ADME relevanten Proteine hohe Bindungsaffinitäten und werden deshalb bezüglich einer Weiterentwicklung zum Wirkstoff als eher ungeeignet eingestuft.
Verbindung 2 zeigt relativ hohe Bindungsaffinitäten gegenüber CYP2D6 und CYP3A4. Für eine Weiterentwicklung würde man deshalb zunächst überprüfen, ob die beobachteten Bindungsaffinitäten gegenüber CYP2D6 und CYP3A4 zu unerwünschten Effekten beim Einsatz als Wirkstoff führen.
Verbindung 4 zeigt relativ hohe Bindungsaffinitäten gegenüber HSA und CYP2D6. Für eine Weiterentwicklung würde man deshalb zunächst überprüfen, ob die beobachteten Bindungsaffinitäten gegenüber HSA und CYP2D6 zu unerwünschten Effekten beim Einsatz als Wirkstoff führen.
Die Verbindung 5 zeigt nur gegen das Protein CYP2D6 eine hohe Bindungsaffinität. Für eine Weiterentwicklung würde man deshalb zunächst überprüfen, ob die sehr starke Bindungsaffinität gegenüber CYP2D6 zu unerwünschten Effekten beim Einsatz als Wirkstoff führt.
Verbindung 6 zeigt relativ hohe Bindungsaffinitäten gegenüber HSA und eine mittlere Bindungsaffinität gegenüber CYP2D6. Für eine Weiterentwicklung würde man deshalb zunächst überprüfen, ob die beobachteten Bindungsaffinitäten gegenüber HSA und CYP2D6 zu unerwünschten Effekten beim Einsatz als Wirkstoff führen.
Verbindung 7 zeigt relativ hohe Bindungsaffinitäten gegenüber HSA. Für eine Weiterentwicklung würde man deshalb zunächst überprüfen, ob die beobachtete Bindungsaffinität gegenüber HSA zu unerwünschten Effekten beim Einsatz als Wirkstoff führen.
Verbindung 8 zeigt relativ hohe Bindungsaffinitäten gegenüber AAG. Für eine Weiterentwicklung würde man deshalb zunächst überprüfen, ob die beobachteten Bindungsaffinitäten gegenüber AAG zu unerwünschten Effekten beim Einsatz als Wirkstoff führen.
Die Verbindungen 9 und 10 zeigen keine bis sehr schwache Bindung gegenüber allen vier analysierten Proteinen und würden deshalb als unkritisch für eine Weiterentwicklung angesehen werden. Somit kann diesen Verbindungen gute ADME Eigenschaften zugeordnet werden.

## Patentansprüche

1. Verfahren zur Analyse der Interaktion von Wirkstoffvorstufen mit ADME relevanten Biomolekülen, die Schritte umfassend
(a) Immobilisierung einer Vielzahl von Wirkstoffvorstufen auf einem festen Träger in einem zweidimensionalen Array;
(b) Inkontaktbringen der Wirkstoffvorstufen mit einem ADME relevanten Biomolekül;
(c) Bestimmung der Bindungsstärken zwischen immobilisierten Wirkstoffvorstufen und ADME relevantem Biomolekül;
(d) Klassifizierung der Wirkstoffvorstufen nach ihrer Bindungsstärke und Zuordnung einer ADME Eigenschaft.

2. Verfahren nach Anspruch 1, **dadurch gekennzeichnet, dass** das Verfahren zusätzlich den Schritt umfasst:
(e) Wiederholung von Schritt (b) bis (d) mit unterschiedlichen ADME relevanten Biomolekülen, um ein ADME Profil der Wirkstoffvorstufen zu erstellen.

3. Verfahren nach Anspruch 1 oder 2, **dadurch gekennzeichnet, daß** die Bestimmung der Bindungsstärken auf Gleichgewichtsmessungen beruhen.

4. Verfahren nach einem oder mehreren der Ansprüche 1 bis 3, **dadurch gekennzeichnet, daß** für die Messungen zur Bestimmung der Bindungsstärken die Wirkstoffvorstufen und ADME relevanten Biomoleküle jeweils nur in einer einzigen Konzentration bereitgestellt werden.

5. Verfahren nach einem oder mehreren der Ansprüche 1 bis 4, **dadurch gekennzeichnet, dass** es sich um eine ADME Eigenschaft aus den Bereichen Absorption, Distribution, Metabolismus oder Exkretion handelt.

6. Verfahren nach einem oder mehreren der Ansprüche 1 bis 5, **dadurch gekennzeichnet, daß** das ADME relevante Biomolekül ausgewählt wird aus der Gruppe bestehend aus Plasmaproteinen, metabolischen Enzymen, CYP 450 Enzymen oder Transportproteinen.

7. Verfahren nach einem oder mehreren der Ansprüche 1 bis 6, **dadurch gekennzeichnet, dass** ein oder mehrere Standardliganden auf dem Array immobilisiert werden.

8. Verfahren nach einem oder mehreren der Ansprüche 1 bis 7, **dadurch gekennzeichnet, daß** die Wirkstoffvorstufen sogenannte kleine organische Moleküle sind.

9. Verfahren nach einem oder mehreren Ansprüchen 1 bis 8, **dadurch gekennzeichnet, daß** mindestens 100, bevorzugt 1000, besonders bevorzugt mindestens 5000 verschiedene Wirkstoffvorstufen immobilisiert werden.

10. Verfahren nach einem oder mehreren der Ansprüche 1 bis 9, **dadurch gekennzeichnet, daß** die Identität einer jeden Wirkstoffvorstufe durch ihre Position auf dem Array erfolgen kann.

11. Verfahren nach einem oder mehreren der Ansprüche 1 bis 10, **dadurch gekennzeichnet, daß** die Immobilisierung der Wirkstoffvorstufen auf dem festen Träger mittels einer organischen Zwischenschicht eine selbstassemblierende Monolage umfassend erfolgt.

12. Verfahren nach Anspruch 11, **dadurch gekennzeichnet, daß** die organische Zwischenschicht polymerfrei ist.

13. Verfahren nach Anspruch 11 oder 12, **dadurch gekennzeichnet, daß** die Monolage Ankermoleküle und gegebenenfalls Verdünnermoleküle umfaßt.

14. Verfahren nach einem oder mehreren der Ansprüche 1 bis 13, **dadurch gekennzeichnet, daß** die Detektion parallel verläuft.

15. Verfahren nach Anspruch 14, **dadurch gekennzeichnet, daß** zur Detektion die Oberflächenplasmonenresonanz (SPR) verwendet wird.

16. Meßsystem umfassend
(e) einen festen Träger auf dem eine Vielzahl von unterschiedlichen Wirkstoffvorstufen in einem zweidimensionalen Array immobilisiert sind,
(f) ein an mindestens einer Wirkstoffvorstufe gebundenes ADME relevantes Biomolekül und
(g) einen Detektor.

17. Verwendung eines Verfahrens nach einem oder mehreren der Ansprüche 1 bis 15 oder eines Meßsystems nach Anspruch 16 zur Klassifizierung oder zum Erstellen von ADME Profilen von Wirkstoffvorstufen.
